# EUROPÄISCHE PATENTANMELDUNG

(11) **EP 2 108 364 A1**
(43) Veröffentlichungstag der Anmeldung: **14.10.2009**
(21) Anmeldenummer: 09157622.3
(22) Anmeldetag: 08.04.2009
(51) Int. Cl.: A61K 9/16, A61K 9/00

(54) **Wasserfreie Wirkstoffträgermatrix für hydrophile Wirkstoffe**

(30) Priorität: 09.04.2008 DE 102008018107
(71) Anmelder: ROVI Cosmetics International GmbH, 36381 Schlüchtern (DE)
(72) Erfinder: Blume, Gabriele, 36396, Steinau an der Straße (DE); Teichmüller, Dirk, 63589, Linsengericht-Geislitz (DE)
(74) Vertreter: WSL Patentanwälte

(57) **Zusammenfassung**

Um einen verbesserten Wirkstoffträger bereitzustellen, mit dessen Hilfe hydrophile Wirkstoffe einfach und stabil wasserfrei formuliert werden können, wird erfindungsgemäß eine kosmetische oder dermatologische Zusammensetzung mit einer wasserfreien Wirkstoffträgermatrix und wenigstens einem in dieser Wirkstoffträgermatrix eingeschlossenen hydrophilen Wirkstoff vorgeschlagen, wobei die Zusammensetzung
a) 60 bis 90 Gew.-% Wachs, wobei der Wachsanteil bezogen auf das Gewicht der gesamten Zusammensetzung
i) 5 bis 20 Gew.-% Carnaubawachs und
ii) 20 bis 50 Gew.-% Jojobawachs umfaßt,

b) 5 bis 10 Gew.-% wenigstens einen nicht wasserlöslichen und nicht oberflächenaktiven polaren Ester, wobei der wenigstens eine polare Ester aus einer Glycerin-, Pentaerythritol- oder Sorbitoleinheit besteht, die in wenigstens zwei Positionen mit Polyoxyalkylketten verethert ist, und wobei die wenigstens zwei Polyoxyalkylketten mit Fettsäuren verestert sind,
c) wahlweise bis zu 10 Gew.-% wenigstens einen Hilfsstoff und
d) 1 bis 20 Gew.-% wenigstens einen hydrophilen Wirkstoff

enthält, wobei die Komponenten a) bis c) die Wirkstoffträgermatrix bilden.

## Beschreibung

Die vorliegende Erfindung betrifft eine kosmetische oder dermatologische Zusammensetzung mit einer wasserfreien Wirkstoffträgermatrix und wenigstens einem in dieser Wirkstoffträgermatrix eingeschlossenen hydrophilen Wirkstoff, wobei die Wirkstoffträgermatrix einen Anteil an pflanzlichem Wachs aufweist.

Weiterhin betrifft die vorliegende Anmeldung eine kosmetische oder dermatologische Zubereitung mit einer wasserhaltigen Zubereitungsbasis und einer in dieser Zubereitungsbasis suspendierten Zusammensetzung mit einer wasserfreien Wirkstoffträgermatrix und wenigstens einem in dieser Wirkstoffträgermatrix eingeschlossenen hydrophilen Wirkstoff, wobei die Wirkstoffträgermatrix einen Anteil an pflanzlichem Wachs aufweist.

Ferner wird ein Verfahren zur Herstellung einer Zusammensetzung mit einer wasserfreien Wirkstoffträgermatrix und wenigstens einem in dieser Wirkstoffträgermatrix eingeschlossenen hydrophilen Wirkstoff beschrieben, wobei die Wirkstoffträgermatrix einen Anteil an pflanzlichem Wachs aufweist.

Viele kosmetisch oder dermatologisch relevante Wirkstoffe bzw. Arzneistoffe sind hydrophil. Für solche hydrophilen Wirkstoffe bzw. Arzneistoffe sind entsprechend geeignete Trägersysteme erforderlich. Zu den bekannten Wirkstoffformulierungen für kosmetische oder dermatologische Zubereitungen, mit deren Hilfe hydrophile Wirkstoffe formuliert werden können, zählen beispielsweise Liposome oder Cyclodextrine, in denen die jeweiligen Wirkstoffe verkapselt werden.

Einige kosmetisch oder pharmazeutisch relevante hydrophile Stoffe können allerdings mit Wasser spontan reagieren bzw. werden in Gegenwart von Wasser chemisch verändert. Aus diesem Grund benötigt man auf dem Gebiet der kosmetischen und dermatologischen Anwendungen geeignete Wirkstoffträger und Zubereitungen, mit deren Hilfe hydrophile Wirkstoffe so formuliert werden können, daß sie während der Herstellung aber auch in der für die Anwendung fertigen Zubereitung vor der Einwirkung von Wasser geschützt sind, damit es zumindest vor der Anwendung der Zubereitung am Körper des Anwenders zu keinen Interaktionen von Wirkstoff und Wasser kommen kann.

Zu diesem Zwecke werden in der Kosmetik 2-Komponentenverpackungen verwendet, bei denen die eine Komponente den wasserfreien hydrophilen Wirkstoff (z.B. dispergiert in Glycerin) enthält und die andere die Formulierungsbasis. Erst beim Auftragen werden die beiden Komponenten zu einer wasserhaltigen Formulierung gemischt. Die separate Verpackung der beiden Komponenten ist jedoch recht aufwendig in der Herstellung. Es besteht daher ein Bedarf nach verbesserten Wirkstoffträgern und Zubereitungen, mit deren Hilfe hydrophile Wirkstoffe einfach im wesentlichen wasserfrei und stabil formuliert werden können, und es ist die Aufgabe der vorliegenden Erfindung entsprechend verbesserte Wirkstoffträger und Zubereitungen bereitzustellen.

Diese Aufgabe wird gelöst durch eine kosmetische oder dermatologische Zusammensetzung mit einer wasserfreien Wirkstoffträgermatrix und wenigstens einem in dieser Wirkstoffträgermatrix eingeschlossenen hydrophilen Wirkstoff, wobei die Zusammensetzung dadurch gekennzeichnet ist, daß sie
a) 60 bis 90 Gew.-% Wachs, wobei der Wachsanteil bezogen auf das Gewicht der gesamten Zusammensetzung
   i) 5 bis 20 Gew.-% Carnaubawachs und
   ii) 20 bis 50 Gew.-% Jojobawachs umfaßt,
b) 5 bis 10 Gew.-% wenigstens einen nicht wasserlöslichen und nicht oberflächenaktiven polaren Ester, wobei der wenigstens eine polare Ester aus einer Glycerin-, Pentaerythritol- oder Sorbitoleinheit besteht, die in wenigstens zwei Positionen mit Polyoxyalkylketten verethert ist, und wobei die wenigstens zwei Polyoxyalkylketten mit Fettsäuren verestert sind,
c) wahlweise bis zu 10 Gew.-% wenigstens einen Hilfsstoff und
d) 1 bis 20 Gew.-% wenigstens einen hydrophilen Wirkstoff enthält, wobei die Komponenten a) bis c) die Wirkstoffträgermatrix bilden.

Der Vorteil der Wirkstoffträgermatrix gemäß der vorliegenden Erfindung besteht darin, daß hydrophile Wirkstoffe in diese Wirkstoffträgermatrix sehr effektiv und stabil eingeschlossen werden können. Besonders vorteilhaft an der erfindungsgemäßen Wirkstoffträgermatrix ist, daß der wenigstens eine hydrophile Wirkstoff auch in ausgesprochen hohen Konzentrationen von der Wirkstoffträgermatrix eingeschlossen werden kann.

Die erfindungsgemäße Zusammensetzung aus Wirkstoffträgermatrix und Wirkstoff gewährleistet, daß wasserempfindliche oder mit Wasser reagierende Wirkstoffe, auch wenn sich die Wirkstoffträgermatrix mit dem darin eingeschlossenen Wirkstoff in einer wasserhaltigen Umgebung befindet, vor der Applikation beim Anwender mit dem in der Umgebung enthaltenen Wasser nicht unmittelbar in Kontakt kommen.

Die erfindungsgemäße Wirstoffträgermatrix kann daher beispielsweise vorteilhaft als Wirkstoffträger für Ascorbinsäure verwendet werden. Ascorbinsäure verliert in Gegenwart von Wasser bereits bei Raumtemperatur seine antioxidative Wirkung. Die vorliegende Erfindung macht es nun möglich, reine Ascorbinsäure für topische Anwendungen so zu formulieren, daß die Wirksamkeit der Ascorbinsäure vor der Anwendung durch Wasser nicht beeinträchtigt werden kann.

Neben der Ascorbinsäure gibt es auch andere empfindliche Antioxidantien, wie z. B. die Polyphenole des grünen Tees, die in wasserhaltigen Formulierungen über die Zeit zu Verfärbungen der Produkte führen können und die ebenfalls schwer zu stabilisieren sind. Auch solche Wirkstoffe können mit Hilfe der vorliegenden Erfindung nun für topische Anwendungen besser formuliert werden.

Manche Wirkstoffe reagieren in Gegenwart von Wasser auch unter beachtlicher Wärmebildung. Diese Generierung von Wärme macht man sich bei einer Reihe von topischen Anwendungen zu Nutze, was allerdings voraussetzt, daß diese Wirkstoffe in der Formulierung nicht schon vor der Anwendung mit Wasser in Kontakt kommen und dabei vorab ihre wärmende Wirkung verlieren. Zu diesem Zwecke kann man solche Wirkstoffe in eine erfindungsgemäße Wirkstoffträgermatrix einschließen. Hierdurch wird erreicht, daß die Wärmebildung nicht zu einem ungewünschten Zeitpunkt, wie z.B. während der Lagerung einer entsprechenden Zubereitung, sondern erst nach der Applikation der Zubereitung (beispielsweise auf die Haut) erfolgt, da der Wirkstoff erst dann mit Wasser in Kontakt kommen kann (siehe unten).

Die Wirkstoffe können beispielsweise durch Verreiben einer die erfindungsgemäße Zusammensetzung enthaltenden Zubereitung mit einer wasserhaltigen Zubereitungsbasis auf der Haut) freigesetzt werden, um an ihren Wirkort zu gelangen. Wird eine solche Zubereitung auf der Haut verrieben, kommt es zur Einlagerung des in der wasserhaltigen Zubereitung enthaltenen Wassers in die Wirkstoffträgermatrix. Diese Wassereinlagerung führt zu einer Umstrukturierung der Wirkstoffträgermatrix, was die Freisetzung des in der Matrix eingeschlossenen Wirkstoffes zur Folge hat. Der Wirkstoff kann daher erst ab dem Moment, in dem er aus der Wirkstoffträgermatrix freigesetzt wird, mit Wasser in Kontakt kommen.

Der Begriff "Wirkstoffträgermatrix" ist für den Fachmann klar und eindeutig. Insbesondere ist hierunter eine Substanz zu verstehen, in die ein Wirkstoff so eingearbeitet werden kann, daß dieser Wirkstoff von der Matrix umgeben wird. Diese Substanz ist erfindungsgemäß aus bestimmten Wachsen, einem Anteil an wenigstens einem nicht wasserlöslichen und nicht oberflächenaktiven polaren Ester und wahlweise wenigstens einem Hilfsstoff zusammengesetzt.

Der Schmelzpunkt der Wirkstoffträgermatrix liegt vorzugsweise bei einer Temperatur von > 50°C. Unter dem Schmelzpunkt der Wirkstoffträgermatrix ist hierbei die Temperatur zu verstehen, die auch als Tropfpunkt bezeichnet wird, d.h. diejenige Temperatur, bei der die Wirkstoffträgermatrix zu fließen beginnt und unter definierten Umständen unter ihrem Eigengewicht von einem definierten Körper abtropft. Die Tropfpunktbestimmung kann nach DIN 51801 (Deutsche Industrienorm) mit einem Tropfpunktgerät nach Ubbelohde erfolgen.

Den Schmelzpunkt der Wirkstoffträgermatrix kann der Fachmann durch geeignete Auswahl der Zusammensetzung des Wachsanteils der Wirkstoffträgermatrix auf den gewünschten Wert einstellen (siehe unten). Darüber hinaus können der Wirkstoffträgermatrix auch einer oder mehrere der unten aufgeführten Hilfsstoffe, wie z.B. Öle, beigefügt werden, um den gewünschten Schmelzpunkt einzustellen.

Erfindungsgemäß ist der Wirkstoff in der Wirkstoffträgermatrix eingeschlossen. Dies ist so zu verstehen, daß der Wirkstoff bei Raumtemperatur oder sogar bei Umgebungstemperaturen von bis zu 35, 40 oder 45°C so in der Wirkstoffträgermatrix eingebettet vorliegt, daß er von dieser vollständig umschlossen ist, so daß der Wirkstoff mit der um die Wirkstoffträgermatrix befindlichen Umgebung nicht unmittelbar in Kontakt kommt. Ein anderer Ausdruck für diesen Zustand ist, daß der Wirkstoff in der Wirkstoffträgermatrix verkapselt vorliegt. Erst wenn sich die Wirkstoffträgermatrix beim Verreiben einer die erfindungsgemäße Zusammensetzung enthaltenden Zubereitung auf der Haut umstrukturiert (s.o.), wird der Wirkstoff aus der Wirkstoffträgermatrix freigesetzt.

Hat ein Wirkstoff, der in die erfindungsgemäße Wirkstoffträgermatrix eingearbeitet wird, Einfluß auf die Schmelzeigenschaften der Zusammensetzung aus Wirkstoffträgermatrix und Wirkstoff, so gelten die oben genannten Schmelz- bzw. Umstrukturierungseigenschaften der Matrix in wässerigem Millieu für die gesamte Zusammensetzung. D.h., daß in diesen Fällen die Auswahl der Zusammensetzung des Wachsanteils und wahlweise auch der Hilfsstoffe vom Fachmann so zu treffen ist, daß die Zusammensetzung aus Wirkstoffträgermatrix und Wirkstoff die geforderten Eigenschaften erfüllt.

Üblicherweise wird der Wirkstoff bei der Ausführung der Erfindung in die erfindungsgemäße Wirkstoffträgermatrix eingearbeitet, indem der Wirkstoff beispielsweise in eine zuvor zubereitete Wirkstoffträgermatrixmasse eingerührt wird. Wenn die Wirkstoffträgermatrixmasse mit dem darin eingearbeiteten Wirkstoff anschließend einem Formgebungsprozeß unterworfen wird, bei dem beispielsweise Partikel erzeugt werden, ist selbstverständlich nicht auszuschließen, daß vereinzelt Wirkstoffmoleküle an der Peripherie dieser Partikel angeordnet sind, so daß diese einzelnen Wirkstoffmoleküle mit der um diese Partikel befindlichen Umgebung in Kontakt kommen können. Da der Anteil der Moleküle, die so an der Peripherie der Partikel angeordnet sind, daß sie theoretisch mit der Umgebung in Kontakt kommen könnten, im Verhältnis zu den in den Partikeln vollständig eingeschlossenen Wirkstoffmolekülen verschwindend gering ist, kann näherungsweise davon ausgegangen werden, daß bei der Ausführung der Erfindung der Wirkstoff im wesentlichen in der Wirkstoffträgermatrix eingeschlossen vorliegt.

Die erfindungsgemäße Wirkstoffträgermatrix hat weiterhin den Vorteil, daß sie in wässerigem bzw. wasserhaltigem Milieu stabil ist, d.h. unter alltäglichen Bedingungen gewährleistet, daß der in ihr enthaltene Wirkstoff vor der Anwendung nicht mit Wasser in Kontakt kommt. Diese Stabilität weist die erfindungsgemäße Wirkstoffträgermatrix jedenfalls bei Raumtemperatur (20 bis 25 °C) auf. Vorzugsweise ist die Wirkstoffträgermatrix auch in dem Temperaturbereich von 20 bis 30°C stabil. Noch bevorzugter ist die Wirkstoffträgermatrix im Bereich von 20 bis 40°C oder gar im Bereich von 20 bis 50°C stabil. Diese Stabilität weist die erfindungsgemäße Wirkstoffträgermatrix vorzugsweise über wenigstens etwa 1 Monat auf, noch bevorzugter über wenigstens 3 Monate. Bei besonders bevorzugten Ausführungsformen weist die erfindungsgemäße Wirkstoffträgermatrix diese Stabilität über wenigstens etwa 6, 12 bzw. 36 Monate auf.

Unter der Bezeichnung "wasserfrei" bzw. "im wesentlichen wasserfrei" wird hier verstanden, daß ein mit diesem Begriff beschriebenes Material im wesentlichen kein freies Wasser enthält. Mit "im wesentlichen" ist in diesem Zusammenhang gemeint, daß nicht ausgeschlossen werden kann, daß in diesem Material trotz allem einzelne Wassermoleküle oder einige wenige Wassermoleküle vereinzelte Mikroeinschlüsse von verschwindend geringen Mengen an Wasser enthalten sind. Vorzugsweise beträgt der Gehalt an Wasser höchstens 0,5 Gew.-% bezogen auf die Wirkstoffträgermatrix. Besonders bevorzugt ist nicht mehr als 0,1 Gew.-% Wasser in der Wirkstoffträgermatrix enthalten. Noch bevorzugter ist nicht mehr als 0,01 Gew.-% Wasser in der Wirkstoffträgermatrix enthalten.

Die vorgenannten Vorteile der erfindungsgemäßen Zusammensetzung werden erzielt, durch eine wasserfreie Wirkstoffträgermatrix, die bezogen auf das Gewicht der gesamten Zusammensetzung einen Anteil von 60 bis 90 Gew.-% Wachs und einen Anteil von 5 bis 10 Gew.-% wenigstens einen nicht wasserlöslichen und nicht oberflächenaktiven polaren Ester, der in der Lage ist, hydrophile Wirkstoffe in seine räumliche Molekülstruktur aufzunehmen, enthält. Die Wachskomponente der Wirkstoffträgermatrix umfaßt dabei bezogen auf das Gewicht der gesamten Zusammensetzung einen Anteil von 5 bis 20 Gew.-% Carnaubawachs und einen Anteil von 20 bis 50 Gew.-% Jojobawachs.

Unter dem Begriff "Wachs" werden hier alle pflanzlichen, tierischen, mineralischen oder synthetischen Wachse und Wachsester verstanden. Unter "Wachsester" versteht der Fachmann die Ester langkettiger Wachssäuren, die wenigstens 22 Kohlenstoffatome aufweisen, mit langkettigen Wachsalkoholen, die etwa 24 - 36 Kohlenstoffatome aufweisen, Triterpenalkoholen oder Steroidalkoholen, sowie Gemische verschiedener Wachsester. Die pflanzlichen, tierischen, mineralischen oder synthetischen Wachse können aus Wachsestern bestehen oder nur einen Anteil von Wachsestern aufweisen bzw. Wachsester als Hauptkomponente aufweisen. Die pflanzlichen, tierischen, mineralischen oder synthetischen Wachse umfassen unabhängig davon, ob sie Wachsester enthalten, auch solche Stoffe oder Stoffgemische, die bei 20°C knetbar, fest bis brüchig hart sind, eine grobe bis feinkristalline Struktur aufweisen und farblich durchscheinend bis opak, aber nicht glasartig sind, und wenn diese Stoffe bzw. das Stoffgemisch bei über 40°C ohne Zersetzung schmelzen, schon wenig oberhalb des Schmelzpunktes niedrigviskos und nicht fadenziehend sind, eine stark temperaturabhängige Konsistenz und Löslichkeit aufweisen sowie unter leichtem Druck polierbar sind.

Es wurde überraschenderweise herausgefunden, daß eine besonders stabile und effektiv vor Wasser schützende Wirkstoffträgermatrix in Verbindung mit den wenigstens einen hydrophilen Wirkstoff tragenden polaren Estern erhalten wird, wenn die Wirkstoffträgermatrix einen Anteil an Carnaubawachs in Verbindung mit einem Anteil an Jojobawachs aufweist.

Carnaubawachs (Cera carnauba) wird aus den Blättern der Carnaubapalme (*Copernicia cerifera*) extrahiert und weist einen hohen Schmelzpunkt (82 bis 87 °C) auf. Durch die erfindungsgemäße Verwendung von Carnaubawachs in der Wirkstoffträgermatrix wird erreicht, daß diese Matrix in üblichen Zubereitungen bzw. Formulierungen stabil ist. Das Wachs besteht aus hydroxylierten Säureestern und Dieestern, die zu einem großen Teil sonnenschützende Eigenschaften aufweisen (UV-Filter). Dies hat den Vorteil, daß die erfindungsgemäß mit Hilfe der polaren Ester in der Carnaubawachs enthaltenden lipophilen Umgebung eingebetteten Wirkstoffe vor der Einwirkung von UV-Strahlung, die die Stabilität der Wirkstoffe möglicherweise beeinträchtigen könnten, geschützt werden.

Die Wachskomponente der Wirkstoffträgermatrix enthält bezogen auf das Gewicht der gesamten Zusammensetzung einen Anteil von 5 bis 20 Gew.-% Carnaubawachs. Vorzugsweise beträgt der Anteil an Carnaubawachs an der erfindungsgemäßen Wirkstoffträgermatrix 5 bis 15 Gew.-%, besonders bevorzugt beträgt der Anteil an Carnaubawachs 5 bis 10 Gew.-%.

Jojobawachs wird aus dem Wüstenstrauch *Simmondsia chinensis* gewonnen und besteht im wesentlichen aus Anteilen an unhydrierten Jojobaölen und/oder Jojobaestern, die teil- bzw. vollkommen hydriert sind. Jojobawachs ist sehr hautfreundlich. Erfindungsgemäß enthält die wasserfreie Wirkstoffträgermatrix einen Anteil von 20 bis 50 Gew.-% Jojobawachs. Vorzugsweise beträgt der Anteil an Jojobawachs 30 bis 50 Gew.-%, besonders bevorzugt beträgt der Anteil an Jojobawachs 40 bis 50 Gew.-%.

Die Erfinder der vorliegenden Anmeldung konnten nachweisen, das Carnaubawachs in Kombination mit Jojobawachsen nach der Applikation auf die Haut die Penetration von Wirkstoffen in tiefere Hautschichten fördert. In einem Versuch mit Lipidpartikeln, die Carnaubawachs, Jojobawachse und den antioxidativ wirkenden Stoff Tocopherol enthielten, konnte gezeigt werden, daß sich die antioxidative Wirkung in den tieferen Hautschichten signifikant stärker entfaltet als bei Tocopherol, welches in Lipidpartikeln verkapselt war, die anders zusammengesetzt waren.

Zusätzlich zu dem Anteil an Carnaubawachs und Jojobawachs kann die erfindungsgemäße Wirkstoffträgermatrix außerdem auch einen Anteil eines weiteren pflanzlichen, tierischen, mineralischen oder synthetischen Wachses enthalten.

Das weitere Wachs oder die weiteren Wachse dient bzw. dienen in erster Linie zur Einstellung der Schmelzeigenschaften bzw. Schmelzpunktes der Wirkstoffträgermatrix. Wird ein Wachs mit niedrigem Schmelzpunkt in die Wirkstoffträgermatrix eingearbeitet, so wird die Wirkstoffträgermatrix insgesamt weicher und schmilzt bei niedrigeren Temperaturen. Wenn ein Wachs mit höherem Schmelzpunkt verwendet wird, so erhält man eine härtere Wirkstoffträgermatrix.

Zu den weiteren Wachsen, die bei der vorliegenden Erfindung zur Anwendung kommen können, zählen insbesondere: Weißtannennadelwachs, Akazienblattwachs, Bienenwachs, hydrolysiertes Bienenwachs, oxidiertes Bienenwachs, PEG-6 Bienenwachs; PEG-8 Bienenwachs, PEG-12 Bienenwachs, PEG-20 Bienenwachs, synthetisches Bienenwachs, Carnaubawachs und saures Carnaubawachs, hydrolysiertes Carnaubawachs, PEG-12 Carnaubawachs, synthetisches Carnaubawachs, Ceresin, Cetylester, Zistrosenblütenwachs (Labdanum), Pomeranzenblütenwachs (Bitterorangenblütenwachs), Orangenblütenwachs, Orangenschalenwachs, Ginsterblütenwachs, Mehlblumenwachs, Schildlauswachs, Candelillawachs, hydrolysiertes Candelillawachs, synthetisches Candelillawachs, Strohblumenwachs (Currykrautwachs), Japanwachs, hydrogeniertes Japanwachs, synthetisches Japanwachs, Jojobabutter, Jojobawachs, Jojobaester, hydrogeniertes Jojobawachs, geschwefeltes Jojobawachs, mikrokristallines Wachs, hydrogeniertes mikrokristallines Wachs, oxidiertes mikrokristallines Wachs, oxidiertes mikrokristallines Wachs - Kaliumsalz, Reiskleienwachs, hydrogeniertes Reiskleienwachs, Lilienblütenwachs, Jasminblütenwachs, Lanolin (Wollwachs), Lavendelblütenwachs, Hennawachs, Nerzwachs, Montanwachs und saures Montanwachs, Wachsmyrtenfruchtwachs, Basilikumwachs (indisch), Ouricurywachs, Ozokerit, Palmkernwachs, Paraffin, Avocadowachs, Mastixblattwachs, Nachthyazinthenblütenwachs, Apfelschalenwachs, Johannisbeerenknospenwachs, Rosenblütenwachs, Rispenrosenblütenwachs, Muskatellersalbeiwachs, Schellack, Gerstentreberwachs, Halfagraswachs, synthetische Wachse, wie z.B. Polyethylenwachse, Polyethylenglykolwachse, Polyethylenglykolesterwachse, wachsartige Silicone, Traubenblattwachs, Zuckerrohrwachs, Walrat, Bürzeldrüsenfett.

Besonders bevorzugt sind Ausführungsformen, bei denen der Wachsanteil auch einen Anteil Japanwachs, Bienenwachs oder Candililawachs umfaßt.

Die nicht wasserlöslichen und nicht oberflächenaktiven polaren Ester, die in der erfindungsgemäßen Wirkstoffträgermatrix enthalten sind, bestehen aus einem Grundkörper, der von einer Glycerin-, einer Pentaerythritol- oder einer Sorbitoleinheit gebildet wird. Dieser Grundkörper ist in wenigstens zwei Positionen mit Polyoxyalkylketten verethert, wobei die Polyoxyalkylketten wiederum mit Fettsäuren verestert sind. Diese polaren Ester haben den Vorteil, daß sie in dem Bereich, in dem die Polyoxyalkylketten vorliegen, hydrophile Verbindungen in ihre dreidimensionale Molekülstruktur aufnehmen können. Die hydrophilen Verbindungen sind damit praktisch "im Inneren" des polaren Esters eingelagert, während das Äußere des polaren Esters durch die apolaren, lipophilen Fettsäurereste gebildet wird. Demnach können diese Ester einschließlich der in ihrem Inneren eingelagerten hydrophilen Substanzen in einer lipophilen Umgebung gelöst werden.

Die Aufnahme der polaren Wirkstoffe in die räumliche Molekülstruktur der polaren Ester erfolgt bei leicht erhöhter Temperatur, vorzugsweise bei 30 bis 50°C, noch bevorzugter bei 40 bis 50°C. Unterhalb dieses Temperaturbereiches sind die hydrophilen Wirkstoffe dann im Inneren des Moleküls fest eingebunden und werden erst bei erhöhten Temperaturen, d.h. vorzugsweise bei 30 bis 50°C, noch bevorzugter bei 40 bis 50°C, wieder freigegeben.

Vorzugsweise sind die Polyoxyalkylketten Polyoxyethylen- oder Polyoxypropylen- oder gemischte Polyoxyethylen-/Polyoxypropylenketten. Die Kettenlänge der Fettsäuren der polaren Ester liegt vorzugsweise in dem Bereich von C8 bis C20. Als besonders vorteilhafte polare Ester haben sich solche herausgestellt, die ausgewählt sind unter Sorbeth-2-hexaoleat, Glycereth-6-tricocoat, Sorbeth-12-hexacocoat, Glycereth-6-trioleat, Glycereth-6-triisostearat, Sorbeth-2-hexaisostearat, Sorbeth-2-hexacaprylat/-caprat, Sorbeth-2-hexalaurat und Hydroxypropyldimethiconisostearat.

Wahlweise kann die Wirkstoffträgermatrix bei der vorliegenden Erfindung 5 bis 10 Gew.-% wenigstens einen Hilfsstoff enthalten. Der Begriff "Hilfsstoff" ist dem Fachmann im Zusammenhang mit Wirkstoffträgermatrices bzw. kosmetischen oder dermatologischen Zusammensetzungen und Zubereitungen bekannt. Insbesondere fallen unter diesen Begriff hier solche Zusatzstoffe, die auf die physikalischen Eigenschaften der Zusammensetzung und deren Stabilität einwirken und/oder der Konservierung der Zubereitung dienen. Beispiele für solche Hilfs- oder Zusatzstoffe sind Tenside, Emulgatoren, Detergenzien, Kieselsäure, Öle, Fette, Alkohole, Gelbildner, Gelatine, anorganische Zusätze, Konservierungsmittel, Bakterizide und Keimhemmer, Verdicker, Elektrolyte oder Komplexbildner.

Zu den Ölen und Fetten, die bei der vorliegenden Erfindung als Hilfsstoff zur Anwendung kommen können, zählen: Rizinusöl (auch hydriert, teilhydriert und/oder acetyliert), Kiwisamenöl, Affenbrotbaumöl, Lichtnussbaumöl, Knoblauchöl, Cashewsamenöl, Engelwurzöl, Erdnussöl, Klettensamenöl, Arganöl, Stachelmohnöl (Argemonöl), Haferkornöl, Dattelsamenöl, Galambutter, Madhucabutter, Paranussöl, Annattosamenöl, Borretschsamenöl, Hirnlipide, Rapssamenöl, Broccolisamenöl, Büffelfett, Butter, Sheabutter und Sheabutteröl, C8-C12 Fettsäuretriglyceride, C10-C18 Fettsäuretriglyceride, C12-C18 Fettsäuretriglyceride, Ringelblumensamenöl, Kapkastanienöl, Tamanuöl (Calophyllumöl), Kameliensamenöl, Teesamenöl, Nangainussöl (Kenarinussöl), Hanfsamenöl, Canolaöl, Caprylsäure/Caprinsäure/Laurinsäure-Triglyceride, Caprylsäure/Caprinsäure/Linolsäure-Triglyceride, Caprylsäure/Caprinsäure/Myristinsäure/Stearinsäure-Triglyceride, Caprylsäure/Caprinsäure/Stearinsäure-Triglyceride, Caprylsäure/Caprinsäure-Triglyceride, Laurinsäure/Palmitinsäure/Ölsäure-Triglyceride, Ölsäure/Linolsäure-Triglyceride, Rizinolsäure/Capronsäure/Caprylsäure/Caprinsäure-Triglyceride, Ölsäure/Palmitinsäure/Laurinsäure/Myristinsäure/Linolsäure-Triglyceride, Papayasamenöl, Chaulmoograsamenöl, Gorlisamenöl, Distelsamenöl, Pekannussöl, Caryocarfruchtöl, Caryocarsamenöl, Rizinusölbenzoat, Kephaline, Quinoasamenöl, Schatullenfarnöl (chinesisch), C10-C40 Isoalkylfettsäuretriglyceride, Wassermelonensamenöl, Kokosnussöl, Dorschleber-/Nerztalgtriglyceride, Dorschleberöl (Lebertran), Kaffeesamenöl, Hiobstränensamenöl, Haselnussöl, Krambesamenöl, Gurkenkernöl, Kürbiskernöl, Karottensamenöl, Hirschtalg/Hirschfett, Samenöl des Wegerichblättrigen Natternkopfs, Palmkernöl, Palmöl, Emuöl, epoxidiertes Sojaöl, Kohlpalmenfruchtöl, Fischleberöl, Fischöl, Kokumbutter, Avelanaöl, Glyceryltriacetylhydroxystearat, Glyceryltriacetylricinoleat, trihydrogeniertes Glycerylrosinat, Sojalipide, Sojaöl, Glycolipide, Glycosphingolipide, Ziegenbutter, Ziegenfett, Baumwollsamenöl, Blutregenalgenöl, Sonnenblumenkernöl, Sanddornöl, Pferdefett, Placentalipide, Mangobutter (afrikanisch), Mangosamenöl, Färberwaidsamenöl (Deutscher Indigo), i-somerisiertes Palmöl, Walnussöl, Schweineschmalz/Schweinefett, Wiesenschaumkrautsamenöl, Leinsamenöl, Samenöl des Echten Steinsamen, Schwammkürbissamenöl, Lupinensamenöl, Bocksdornsamenöl, Macadamianussöl, Mamushi Schlangenöl, Murmeltieröl, Knochenmarklipide, Teebaumöl, Neemsamenöl (Niemsamenöl), Melissensamenöl, Menhadenöl, Milchlipide, Nerzöl, Nonisamenöl, Behensamenöl (Moringaöl), Mortierellaöl, Öl gewonnen durch *Mucor circinelloides*, Perlmuttlipide, Klauenöl/Knochenöl, Lotusblumenöl, Schwarzkümmelöl, Kieselalgenöl, Bacabapalmenfruchtöl, Batauapalmenfruchtöl, Nachtkerzenöl, Olivenfruchtöl, Olivenschalenöl, Oleostearin, Rindernetzlipide, Granatbarschöl (Orange Roughy), Babassupalmensamenöl, Babassuöl, Babassukernöl, Reiskeimöl, Reiskleienöl, Straußenöl, oxidiertes Maisöl, oxidiertes Haselnussöl, Moosbeerensamenöl, Mohnsamenöl, Schlafmohnsamenöl, Mobolapflaumensamenöl, Passionsblumensamenöl, Perillasamenöl, Avocadobutter, Avocadoöl, Flachssamenöl, Korea-Kiefernsamenöl, Bonsai-Kiefernsamenöl, Mädchenkiefernsamenöl, Pfeffersamenöl, Pistazienkernöl, Plazentalipide, Sacha-Inchi-Öl, Pongamsamenöl, Bittermandelkernöl, Mandelöl, Aprikosenkernöl, Süßkirschensamenöl, Sauerkirschenkernöl, Pflaumenkernöl, Wildpflaumenkernöl, Pfirsichkernöl, Granatapfelkernöl, Ilombanussöl, Apfelöl, Apfelsamenöl, Kaninchenfett, Johannisbeerensamenöl, Mongongosamenöl, Rizinussamenöl, Hagebuttenfruchtöl, Hagebuttenkernöl, Moschusrosensamenöl, Wildrosensamenöl, Moltebeerensamenöl, Andenbeerensamenöl, Himbeerensamenöl, Lachsöl, Chiasamenöl, Sandelholzsamenöl, Pfefferbaumöl (peruanisch), Mankettikernöl, Lackbaumsamenöl, Marulabaumsamenöl, Sesamsamenöl, Hailipide, Haileberöl, Salbutter, Seidenraupenöl, Seidenraupenlipide, Hautlipide, Tomatenfruchtöl, Tomatensamenöl, Sojabohnenglyceride, Sphingolipide, Rückenmarklipide, Stinkbaumsamenöl, Tallöl, Talg, Agaröl (pazifisch), Kakaobutter, Nusseibensamenöl, Triarachidin, Tribehenin, Tricaprin, Tricaprylin, Buschtabaköl (australisch), Trierucin, Triethylhexanoin, Triheptanoin, Triheptylundecanoin, Trihydroxymethoxystearin, Trihydroxystearin, Triisononanoin, Triisopalmitin, Triisostearin, Trilaurin, Trilinolein, Trilinolenin, Trimyristin, Triolein, Tripalmitin, Tripalmitolein, Triricinolein, Trisebacin, Tristearin, Triundecanoin, Weizenkleienlipide, Weizenkeimöl, Schildkrötenöl, Kranbeerensamenöl (Moosbeerensamenöl), Blaubeerensamenöl, Preiselbeerensamenöl, Vanillefruchtöl, Vateriatalg (Vateriabutter), Pflanzenöl, Virolafett (Virolanussöl), Traubenkernöl, Maiskeimöl, Maisöl sowie deren hydrogenierte und/oder teilhydrogenierte Derivate.

Die Öle dienen in erster Linie zur Einstellung des Schmelzpunktes der Wirkstoffträgermatrix. Wird ein Öl mit niedrigem Schmelz- bzw. Tropfpunkt in die Wirkstoffträgermatrix eingearbeitet, so wird die Wirkstoffträgermatrix insgesamt weicher und schmilzt bei niedrigeren Temperaturen. Wenn ein Öl mit höherem Schmelzpunkt verwendet wird, so erhält man eine härtere Wirkstoffträgermatrix.

Weiterhin ist es bevorzugt, daß als Hilfsstoff auch ein Anteil an Glycerin, Pentandiol, Phospholipiden (z.B. Phosphatidylcholin) und/oder Silikonöl in der Wirkstoffträgermatrix vorliegt. Der Zusatz dieser Hilfsstoffe kann der erfindungsgemäßen Wirkstoffträgermatrix im Falle von Zubereitungen für topische Anwendungen ein positives Hautgefühl beim Verreiben der Wirkstoffträgermatrix verleihen.

Die erfindungsgemäße Wirkstoffträgermatrix zeichnet sich dadurch aus, daß sie besonders große Mengen an hydrophilem Wirkstoff in sich aufnehmen und stabil tragen kann. Gemäß der vorliegenden Erfindung enthält die Zusammensetzung 1 bis 20 Gew.-% wenigstens einen hydrophilen Wirkstoff, der in der Wirkstoffträgermatrix eingeschlossen vorliegt. Bei einer bevorzugten Ausführungsform der Erfindung liegt der Wirkstoffanteil im Bereich von 5 bis 20 Gew.-%. Liegen mehrere Wirkstoffe vor, so macht die Summe der Wirkstoffe 1 bis 20 Gew.-%, vorzugsweise 5 bis 20 Gew.-% aus. Der Begriff "Wirkstoff" ist dem Fachmann bekannt und ist im Zusammenhang mit dieser Erfindung möglichst breit zu verstehen. Dieser Begriff umfaßt in diesem breiten Sinne alle Stoffe, die bei der Anwendung an einem Lebewesen eine physiologische, antibiotische oder protektive Wirkung entfalten können. Im Zusammenhang mit der erfindungsgemäßen kosmetischen oder dermatologischen Zubereitung handelt es sich entsprechend vorzugsweise um einen in irgendeiner Weise kosmetisch oder dermatologisch relevanten Wirkstoff, d.h. um einen Stoff, der in kosmetischer oder dermatologischer Hinsicht eine positive Wirkung haben kann.

Für den Fachmann ist auch klar, welche Wirkstoffe als hydrophil zu bezeichnen sind. Insbesondere ist ein Wirkstoff gemäß der vorliegenden Erfindung dann als hydrophil zu bezeichnen, wenn dieser Wirkstoff sich gut in Wasser lösen läßt.

Der Wirkstoff kann, ohne daß die Erfindung hierauf in irgendeiner Weise beschränkt ist, beispielsweise ausgewählt sein unter Coffein, Carnithin, Magnesiumsulfat, wasserlöslichen Vitaminen und einem hydrophilen Antioxidans, welches ausgewählt ist unter antioxidativen Aminosäuren oder Peptiden, Polyphenolen und Fruchtextrakten, und deren Derivaten oder Gemischen davon. Weiterhin bevorzugt sind Wirkstoffe, die in der Gegenwart von Wasser Wärme bilden, wie z. B. Salze, und/oder die Durchblutung fördern.

Je nach Anwendung und Wirkstoff ist es bei bestimmten Ausführungsformen bevorzugt, wenn die Zusammensetzung bzw. Zubereitung nur einen Wirkstoff enthält. Bei alternativen Ausführungsformen ist es jedoch bevorzugt, wenn die Zusammensetzung bzw. Zubereitung mehrere Wirkstoffe umfaßt. Der oder die Wirkstoffe können entweder alle in der Wirkstoffträgermatrix eingeschlossen vorliegen oder es können einer oder mehrere Wirkstoffe in der Wirkstoffträgermatrix und darüber hinaus einer oder mehrere Wirkstoffe in der wasserhaltigen Zubereitungsbasis vorliegen. Darüber hinaus können in der Wirkstoffträgermatrix oder in einer die Wirkstoffträgermatrix umgebenden Zubereitungsbasis auch lipophile Wirkstoffe vorliegen.

Selbstverständlich handelt es sich bei allen Komponenten der erfindungsgemäßen Zusammensetzung um kosmetisch oder dermatologisch verträgliche Stoffe. Ein Stoff ist im Sinne dieser Erfindung kosmetisch oder dermatologisch verträglich, wenn er nicht toxisch ist und bei der Mehrzahl der potentiellen Anwender topisch anwendbar ist.

Bei einer Ausführungsform der Erfindung liegt die Wirkstoffträgermatrix in Form von Partikeln vor. Vorzugsweise beträgt der mittlere Durchmesser der Partikel von 0,1 bis 3 mm. Besonders bevorzugt sind relativ kleine Partikel mit einem mittleren Durchmesser von 0,1 bis 1 mm, da solche Partikel schneller schmelzen können und sich daher etwas besser verreiben lassen. Alternativ sind auch relativ große Partikel mit einem mittleren Durchmesser von 1 bis 3 mm besonders bevorzugt, da solche Partikel ein vorteilhaft großes Verhältnis von Volumen zu Oberfläche haben und daher anteilsmäßig deutlich mehr Wirkstoffmoleküle in der Wirkstoffträgermatrix vollständig eingeschlossen sind (siehe oben). Eine weitere alternative Ausführungsform stellt einen optimalen Kompromiß zwischen den beiden zuvor erwähnten vorteilhaften Bereichen dar und ist durch Partikel mit einem mittleren Durchmesser von 0,5 bis 2,0 mm gekennzeichnet.

Neben der oben diskutierten Zusammensetzung aus Wirkstoffträgermatrix und Wirkstoff umfaßt die vorliegende Erfindung auch eine kosmetische oder dermatologische Zubereitung, die eine wasserhaltige Zubereitungsbasis und eine in der Zubereitungsbasis vorliegende erfindungsgemäße Wirkstoffzusammensetzung, wie sie oben diskutiert wird, umfaßt. Vorzugsweise ist die Wirkstoffträgermatrix in der Zubereitungsbasis suspendiert. Bei der erfindungsgemäßen Zubereitung liegt der wenigstens eine Wirkstoff im Inneren der Wirkstoffträgermatrix so eingeschlossen vor, daß vor der kosmetischen oder dermatologischen Anwendung der Zubereitung kein unmittelbarer Kontakt des Wirkstoffs mit der Zubereitungsbasis besteht.

Die kosmetische oder dermatologische Zubereitung gemäß der vorliegenden Erfindung ist für die topische Anwendung bestimmt. Wird diese Zubereitung bei der topischen Anwendung beispielsweise auf der Haut verstrichen oder verrieben, so vermischt sich der Wasseranteil der wasserhaltigen Zubereitungsbasis mit der aufgrund der Wärme der Haut oder der beim Verreiben zusätzlich entstehenden Wärme allmählich schmelzenden Wirkstoffträgermatrix. Hierbei kommt es zur Einlagerung des in der wasserhaltigen Zubereitung enthaltenen Wassers in die Wirkstoffträgermatrix. Diese Wassereinlagerung führt zu einer Umstrukturierung der Wirkstoffträgermatrix, was die Freisetzung des in der Matrix eingeschlossenen Wirkstoffes zur Folge hat.

Demnach ist die kosmetische oder dermatologische Zubereitung gemäß der Erfindung vorzugsweise **dadurch gekennzeichnet, daß** sich die wasserfreie Wirkstoffträgermatrix in der wasserhaltigen Zubereitungsbasis nach Verreiben der Wirkstoffträgermatrix so umstrukturiert, daß sie den Wirkstoff freisetzt. Anders ausgedrückt ist die kosmetische oder dermatologische Zubereitung gemäß der Erfindung vorzugsweise **dadurch gekennzeichnet, daß** die wasserfreie Wirkstoffträgermatrix nach topischer Applikation der Zubereitung auf der Haut den Wirkstoff freisetzt, damit dieser in die Haut penetrieren kann.

Vorzugsweise handelt es sich bei der wasserhaltigen Zubereitungsbasis um ein Gel, eine Salbe, eine Paste, eine Lotion oder eine Creme.

Es wird weiterhin ein Verfahren zur Herstellung einer erfindungsgemäßen Wirkstoffträgermatrix und/oder einer eine solche Wirkstoffträgermatrix enthaltenden erfindungsgemäßen Zubereitung beansprucht, bei dem man zunächst den Wachsanteil wahlweise gemeinsam mit einem Hilfsstoff gemäß der vorliegenden Erfindung schmilzt. Des weiteren wird bei diesem Verfahren der wenigstens eine Wirkstoff in dem wenigstens einen nicht wasserlöslichen und nicht oberflächenaktiven polaren Ester gemäß der vorliegenden Erfindung dispergiert. Schließlich wird die Dispersion des wenigstens einen Wirkstoffs in dem wenigstens einen polaren Ester in die Schmelze aus Wachs und Öl eingearbeitet, bis eine homogene Mischung entstanden ist. Die auf diese Weise erhaltene, einen Wirkstoff tragende Wirkstoffträgermatrix kann dann in geeigneter Weise weiterverarbeitet werden. Vorzugsweise werden aus dem Gemisch von Schmelze und Dispersion unmittelbar Partikel oder Granulate erzeugt.

Das homogene Gemisch von Schmelze und Dispersion, die daraus erzeugten Partikel oder die daraus erzeugten Granulate können zunächst in dieser Form aufbewahrt oder unmittelbar im Anschluß an deren Erzeugung in die gewünschte kosmetische oder dermatologische Zubereitung formuliert werden.

Zu den geeigneten Verfahren zur Herstellung von Partikeln oder Granulaten der Wirkstoffträgermatrix zählen Sprüherstarrungs- und Sprühgranulationsverfahren sowie Sprühtrocknungsverfahren unter Verwendung eines Rotating Disc Atomizers sowie das von der Firma Brace GmbH in Alzenau etablierte Mikrokugelverfahren (DE 196 17 924) und die von der Firma GeniaLab GmbH in Braunschweig etablierte Jet Cutter-Technologie.

Für Zwecke der ursprünglichen Offenbarung wird darauf hingewiesen, daß sämtliche Merkmale, wie sie sich aus der vorliegenden Beschreibung und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

Weitere Merkmale, Merkmalskombinationen und Vorteile der vorliegenden Erfindung werden aus den folgenden nicht beschränkenden Beispielen ersichtlich.

### Beispiele

### Beispiel 1 - Lipidmikropartikel mit reiner Ascorbinsäure:

5 Gew.-% Carnaubawachs
20 Gew.-% Jojobaester 60 (PNJ 60)
20 Gew.-% Jojobaester 70 (PNJ 70)
35 Gew.-% Japanwachs
10 Gew.-% Ascorbinsäure
5 Gew.-% Glycereth-6-tricocoat
2,5 Gew.-% Phospholipide (> 70 Gew.-% Phosphatidylcholin)
2,5 Gew.-% 1,2-Pentandiol
   Eine Lagerung dieser Partikel in einem klaren Hydrogel (Wasser und Carbomer) ergab in einem Zeitraum von 14 Tagen bei 45°C keine Verfärbung des Gels. Dies läßt auf eine hohe Stabilität der Partikel schließen, da die Kontrolle (freie Ascorbinsäure im Gel) zu einer gelb-bräunlich Verfärbung der Formulierung führte.

### Beispiel 2 - Lipidmikropartikel zu Massagezwecken:

5 Gew.-% Carnaubawachs
10 Gew.-% Jojobaester 60 (PNJ 60)
25 Gew.-% Jojobaester 70 (PNJ 70)
30 Gew.-% Japanwachs
10 Gew.-% Coffein
5 Gew.-% L-Carnitin
5 Gew.-% Vanillylbutylether (wärmend und durchblutungsfördernd)
5 Gew.-% Glycereth-6-tricocoate
2,5 Gew.-% Phospholipide (> 75 Gew.-% Phosphatidylcholin)
2,5 Gew.-% 1,2-Pentandiol
   Um eine gute Anticellulitewirkung zu erzielen, müssen a) mindestens 2 % Coffein in der Fertigkosmetik eingesetzt werden und muß b) Coffein in hinreichendem Maße in die Haut penetrieren. Die erfindungsgemäße Zubereitung gemäß Beispiel 2 erfüllt beide Anforderungen, nämlich eine hohe Konzentration an Coffein und eine überraschend gute Penetration des Coffeins in die Haut.

## Patentansprüche

1. Kosmetische oder dermatologische Zusammensetzung mit einer wasserfreien Wirkstoffträgermatrix und wenigstens einem in dieser Wirkstoffträgermatrix eingeschlossenen hydrophilen Wirkstoff, wobei die Zusammensetzung **dadurch gekennzeichnet ist, daß** sie
a) 60 bis 90 Gew.-% Wachs, wobei der Wachsanteil bezogen auf das Gewicht der gesamten Zusammensetzung
i) 5 bis 20 Gew.-% Carnaubawachs und
ii) 20 bis 50 Gew.-% Jojobawachs umfaßt,
b) 5 bis 10 Gew.-% wenigstens einen nicht wasserlöslichen und nicht oberflächenaktiven polaren Ester, wobei der wenigstens eine polare Ester aus einer Glycerin-, Pentaerythritol- oder Sorbitoleinheit besteht, die in wenigstens zwei Positionen mit Polyoxyalkylketten verethert ist, und wobei die wenigstens zwei Polyoxyalkylketten mit Fettsäuren verestert sind,
c) wahlweise bis zu 10 Gew.-% wenigstens einen Hilfsstoff und
d) 1 bis 20 Gew.-% wenigstens einen hydrophilen Wirkstoff
enthält, wobei die Komponenten a) bis c) die Wirkstoffträgermatrix bilden.

2. Zusammensetzung nach Anspruch 1, **dadurch gekennzeichnet, daß** die Polyoxyalkylketten Polyoxyethylen- oder Polyoxypropylen- oder gemischte Polyoxyethylen-/Polyoxypropylenketten sind.

3. Zusammensetzung nach einem der Ansprüche 1 und 2, **dadurch gekennzeichnet, daß** die Kettenlänge der Fettsäuren des wenigstens einen polaren Esters C8 bis C20 beträgt.

4. Zusammensetzung nach einem der Ansprüche 1 bis 3, **dadurch gekennzeichnet, daß** der wenigstens eine polare Ester ausgewählt ist unter Sorbeth-2-hexaoleat, Glycereth-6-tricocoat, Sorbeth-12-hexacocoat, Glycereth-6-trioleat, Glycereth-6-triisostearat, Sorbeth-2-hexaisostearat, Sorbeth-2-hexacaprylat/-caprat, Sorbeth-2-hexalaurat und Hydroxypropyldimethiconisostearat.

5. Zusammensetzung nach einem der Ansprüche 1 bis 4, **dadurch gekennzeichnet, daß** der Wachsanteil a) der Wirkstoffträgermatrix bezogen auf das Gewicht der gesamten Zusammensetzung 20 bis 40 Gew.-% Japanwachs umfaßt.

6. Zusammensetzung nach einem der Ansprüche 1 bis 5, **dadurch gekennzeichnet, daß** die Wirkstoffträgermatrix in Form von Partikeln vorliegt.

7. Zusammensetzung nach Anspruch 6, **dadurch gekennzeichnet, daß** der mittlere Durchmesser der Partikel im Bereich von 0,5 bis 3 mm liegt.

8. Zusammensetzung nach einem der Ansprüche 1 bis 7, **dadurch gekennzeichnet, daß** der wenigstens eine Wirkstoff ausgewählt ist unter Coffein, Carnithin, Magnesiumsulfat, Vanillylbutylether, wasserlöslichen Vitaminen und einem hydrophilen Antioxidans, welches ausgewählt ist unter antioxidativen Aminosäuren oder Peptiden, Polyphenolen und Fruchtextrakten, und deren Derivaten oder Gemischen davon sowie Stoffen, die in der Gegenwart von Wasser Wärme bilden und durchblutungsfördernden Stoffen.

9. Kosmetische oder dermatologische Zubereitung umfassend
a) eine wasserhaltige Zubereitungsbasis,
b) eine in der Zubereitungsbasis suspendierte Wirkstoffzusammensetzung nach einem der Ansprüche 1 bis 8,
wobei der wenigstens eine Wirkstoff im Inneren der Wirkstoffträgermatrix so eingeschlossen vorliegt, daß vor der kosmetischen oder dermatologischen Anwendung der Zubereitung kein unmittelbarer Kontakt des Wirkstoffs mit der Zubereitungsbasis besteht und wobei die wasserfreie Wirkstoffträgermatrix bei der topischen Applikation auf der Haut den Wirkstoff freisetzt.

10. Zubereitung nach Anspruch 9, **dadurch gekennzeichnet, daß** die Zubereitung eine Salbe, ein Hydrogel, eine Paste, eine Lotion oder eine Creme ist.

11. Verfahren zur Herstellung einer Zusammensetzung nach einem der Ansprüche 1 bis 8 oder einer Zubereitung nach einem der Ansprüche 9 oder 10, bei dem man
a) die Wachskomponenten der Wirkstoffträgermatrix gemeinsam mit den übrigen Komponenten der Wirkstoffträgermatrix schmilzt,
b) den wenigstens einen Wirkstoff in dem wenigstens einen nicht wasserlöslichen und nicht oberflächenaktiven polaren Ester dispergiert,
c) die Dispersion aus b) in die Schmelze aus a) einarbeitet und
d) ein homogenes Gemisch von Schmelze und Dispersion erzeugt.

12. Verfahren nach Anspruch 11, **dadurch gekennzeichnet, daß** man aus dem Gemisch von Schmelze und Dispersion Partikel oder Granulate erzeugt.
